# EUROPÄISCHE PATENTSCHRIFT

(11) **EP 3 501 463 B1**
(45) Veröffentlichungstag und Bekanntmachung des Hinweises auf die Patenterteilung: **13.01.2021**
(21) Anmeldenummer: 17209073.0
(22) Anmeldetag: 20.12.2017
(51) Int. Cl.: A61F 9/008

(54) **OPHTHALMOLOGISCHE VORRICHTUNG ZUM BEARBEITEN VON AUGENGEWEBE MITTELS EINES GEPULSTEN LASERSTRAHLS**
OPHTHALMOLOGICAL DEVICE FOR TREATING EYE TISSUE USING A PULSED LASER BEAM
DISPOSITIF OPHTALMOLOGIQUE DE TRAITEMENT DE TISSU OCULAIRE À L'AIDE D'UN RAYON LASER D'USINAGE PULSÉ

(43) Veröffentlichungstag der Anmeldung: 26.06.2019
(73) Patentinhaber: Ziemer Ophthalmic Systems AG, 2562 Port (CH)
(72) Erfinder: RATHJEN, Christian, 28197 Bremen (DE)
(74) Vertreter: Rentsch Partner AG

(56) Entgegenhaltungen:
- EP-A1- 1 731 120
- EP-A1- 2 596 775
- US-A1- 2011 028 958

## Beschreibung

### Technisches Gebiet

Die vorliegende Erfindung betrifft eine ophthalmologische Vorrichtung zum Bearbeiten von Augengewebe mittels eines gepulsten Laserstrahls. Die vorliegende Erfindung betrifft insbesondere eine ophthalmologische Vorrichtung zur Gewebevolumenbearbeitung im Augengewebe mittels eines gepulsten Laserstrahls.

### Stand der Technik

Zum Bearbeiten von Augengewebe mittels eines Laserstrahls wird ein Bearbeitungsbereich mit Laserpulsen gescannt (abgetastet), indem der gepulste Laserstrahl mittels geeigneter Scannersysteme (Ablenkvorrichtungen entlang einer Scannbearbeitungslinie geführt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind und den gepulste Laserstrahl in ein oder zwei Scannrichtungen (Abtastrichtungen) ablenken, beispielsweise mit Galvanoscannern, Piezoscannern, Polygonscannern oder Resonanzscannern.

In US 7,621,637 wird eine ophthalmologische Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels einer mechanisch bewegten Projektionsoptik einen Arbeitsbereich gemäss einem Scannmuster abfährt. Die im Vergleich zur mechanischen Bewegung viel schnellere Ablenkung in der Scannrichtung wird im Applikationskopf auf die mechanische Bewegung der Projektionsoptik und somit auf dessen Scannmuster überlagert. Ein Fast-Scannersystem in der Basisstation ermöglicht eine feine Bewegung der Laserpulse (Microscann), welche auf das Scannmuster der beweglichen Projektionsoptik überlagert wird, die einen grossen Bearbeitungsbereich abdeckt, beispielsweise das gesamte Auge

In EP 1 731 120 wird ein ophthalmologisches System beschrieben, das einen Hauptscanner (XY- oder XYZ-Scanner) sowie dem Hauptscanner vorgeschaltete Scannvorrichtung, aufweist. Die Scannvorrichtung erzeugt die eine zusätzliche Scannbewegungskomponente und weist eine höhere Scanngeschwindigkeit als der Hauptscanner auf.

Derartige bekannte Systeme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei Anwendungen, in denen nicht nur flächige Gewebeschnitte in einer im Wesentlichen horizontal ausgerichteten Bearbeitungsfläche auf einer gemeinsamen Fokalfläche ausgeführt werden, sondern in denen Gewebevolumen im Augengewebe bearbeitet werden sollen, die sich über unterschiedliche Fokushöhen ausdehnen und grösser als das Bearbeitungsfeld der Fokussieroptik sind, erweist sich das vertikale Verfahren der Projektionsoptik oder eines Zoom-Systems für eine vertikale Veränderung des Fokus und damit der Bearbeitungshöhe während der Bearbeitung des Augengewebes als zu langsam, im Vergleich zur horizontalen Bearbeitungsgeschwindigkeit. Des Weiteren führen nebeneinanderliegende Bearbeitungsvolumina zu Abschattungen und damit zu unvollständigen Volumenbearbeitungen.

### Darstellung der Erfindung

Es ist eine Aufgabe der vorliegenden Erfindung eine ophthalmologische Vorrichtung zum Bearbeiten von Augengewebe mittels eines gepulsten Laserstrahls vorzuschlagen, welche zumindest einige Nachteile der bekannten Systeme nicht aufweist. Es ist insbesondere eine Aufgabe der vorliegenden Erfindung eine ophthalmologische Vorrichtung zum Bearbeiten von Augengewebe mittels eines gepulsten Laserstrahls vorzuschlagen, welche eine effiziente und präzise Gewebevolumenbearbeitung im Augengewebe ermöglicht.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zum Bearbeiten von Augengewebe umfasst eine Laserquelle, die eingerichtet ist, einen gepulsten Laserstrahl zu erzeugen, ein Scannersystem, das eingerichtet ist, den gepulsten Laserstrahl mit einer Bearbeitungsgeschwindigkeit im Augengewebe entlang einer Scannbearbeitungslinie zu lenken, und eine erste dem Scannersystem vorgeschaltete Scannvorrichtung, welche eingerichtet ist, den gepulsten Laserstrahl zur Erzeugung einer ersten zusätzlichen Scannbewegungskomponente in einer ersten Scannrichtung abzulenken mit einer im Vergleich zur Bearbeitungsgeschwindigkeit wesentlich höheren ersten Scanngeschwindigkeit, so dass die erste zusätzliche Scannbewegungskomponente der Scannbearbeitungslinie überlagert wird.

Die oben genannten Ziele werden durch die vorliegende Erfindung insbesondere dadurch erreicht, dass die ophthalmologische Vorrichtung zudem eine zweite dem Scannersystem vorgeschaltete Scannvorrichtung umfasst, welche eingerichtet ist, den gepulsten Laserstrahl zur Erzeugung einer zweiten zusätzlichen Scannbewegungskomponente in einer zweiten Scannrichtung abzulenken, die abgewinkelt zur ersten Scannrichtung der ersten zusätzlichen Scannbewegungskomponente verläuft, mit einer im Vergleich zur ersten Scanngeschwindigkeit wesentlich höheren zweiten Scanngeschwindigkeit, so dass die zweite zusätzliche Scannbewegungskomponente der ersten zusätzlichen Scannbewegungskomponente überlagert wird und durch die Ablenkung des gepulsten Laserstrahls mit der ersten zusätzlichen Scannbewegungskomponente in der ersten Scannrichtung und der überlagerten zweiten zusätzlichen Scannbewegungskomponente in der zweiten Scannrichtung ein Scanngebiet bearbeitet wird, und einen Schaltkreis, welcher eingerichtet ist, das Scannersystem derart mit der ersten Scannvorrichtung und der zweiten Scannvorrichtung synchronisiert zu steuern, dass das Scanngebiet durch das Scannersystem entlang der Scannbearbeitungslinie verfahren wird und im Augengewebe eine Gewebevolumenbearbeitung bewirkt wird.

In einer Ausführungsvariante sind das Scannersystem, die erste Scannvorrichtung und die zweite Scannvorrichtung derart ausgeführt, dass das durch die erste zusätzliche Scannbewegungskomponente und die zweite zusätzliche Scannbewegungskomponente definierte Scanngebiet und die Bearbeitungslinie quer zueinander verlaufen, und das Scanngebiet durch das Scannersystem zur Volumenbearbeitung im Augengewebe entlang der Scannbearbeitungslinie verfahren wird.

Insbesondere in einer Ausführungsvariante, in welcher das Scannersystem ein Antriebssystem umfasst, das mit der Fokussieroptik gekoppelt ist, die den Laserstrahl ins Augengewebe fokussiert, und das eingerichtet ist, die Fokussieroptik in Bearbeitungsrichtungen einer Bearbeitungsebene zu verfahren, welche normal zur optischen Achse der Fokussieroptik angeordnet ist, ermöglicht das Verfahren des Scanngebiets entlang der Scannbearbeitungslinie durch Verfahrbewegung der Fokussieroptik Gewebevolumenbearbeitung im Auge in einem grossen Bereich präzise und abschattungsfrei durchzuführen, beispielsweise im gesamten Augenbereich.

In einer Ausführungsvariante umfasst das Scannersystem eine Fokussiervorrichtung und ist eingerichtet, einen Fokus des gepulsten Laserstrahls im Augengewebe mit einer in Projektionsrichtung verlaufenden Richtungskomponente zu bewegen, um das durch die erste zusätzliche Scannbewegungskomponente und die zweite zusätzliche Scannbewegungskomponente definierte Scanngebiet zur Volumenbearbeitung im Augengewebe mit einer in Projektionsrichtung verlaufenden Richtungskomponente zu verfahren. Dieser Sonderfall, mit vertikalem Verfahren des Scanngebiets, ermöglicht eine pfostenförmige Bearbeitung von Augengewebe.

In einer Ausführungsvariante umfasst die ophthalmologische Vorrichtung ein der ersten Scannvorrichtung und der zweiten Scannvorrichtung nachgeschaltetes Rotationselement, welches eingerichtet ist, das durch die erste zusätzliche Scannbewegungskomponente und die zweite zusätzliche Scannbewegungskomponente definierte Scanngebiet um eine optische Übertragungsachse zu drehen. Der Schaltkreis ist zudem eingerichtet, das Rotationselement derart zu steuern, dass das Rotationselement das Scanngebiet mit einem von der Scannbearbeitungslinie abhängigen Drehwinkel um die optische Übertragungsachse dreht, so dass das Scanngebiet zur Gewebevolumenbearbeitung im Augengewebe mit einer einstellbaren Ausrichtung zur Scannbearbeitungslinie entlang der Scannbearbeitungslinie verfahren wird. Durch die Drehung des Scanngebiets kann dessen Ausrichtung während des Verfahrens des Scanngebiets flexibel auch an gekrümmte, kreisförmige, spiralförmige oder spiralarmförmige Bearbeitungslinien angepasst werden.

In einer Ausführungsvariante umfasst die ophthalmologische Vorrichtung einen Divergenzmodulator, welcher eingerichtet ist, das durch die erste zusätzliche Scannbewegungskomponente und die zweite zusätzliche Scannbewegungskomponente definierte Scanngebiet bezüglich einer Referenzebene zu verkippen. Der Schaltkreis ist zudem eingerichtet, den Divergenzmodulator derart zu steuern, dass der Divergenzmodulator das Scanngebiet mit einem einstellbaren Verkippungswinkel zur Referenzebene verkippt, so dass das Scanngebiet zur Gewebevolumenbearbeitung im Augengewebe mit dem einstellbaren Verkippungswinkel zur Referenzebene entlang der Scannbearbeitungslinie verfahren wird. Durch die Verkippung des Scanngebiets kann durch Verfahren des verkippten Scanngebiets ohne vertikale Bewegung der Fokussiervorrichtung respektive der Fokussieroptik eine Volumenbearbeitung erzeugt werden.

In einer Ausführungsvariante umfasst der Divergenzmodulator mindestens ein optisches Element, welches der ersten Scannvorrichtung und der zweiten Scannvorrichtung nachgeschaltet ist und welches eingerichtet ist, zur Verkippung des Scanngebiets eine von der ersten zusätzlichen Scannbewegungskomponente und der zweiten zusätzlichen Scannbewegungskomponente abhängige Divergenz des Laserstrahls zu erzeugen.

In einer Ausführungsvariante ist das optische Element um eine optische Übertragungsachse drehbar ausgebildet.

In einer Ausführungsvariante umfasst das optische Element eine Keilplatte, ein Prisma, eine Linse, ein diffraktives optisches Element und/oder einen asphärischen Spiegel.

In einer Ausführungsvariante ist der Divergenzmodulator der ersten Scannvorrichtung vorgeschaltet und ist eingerichtet, zur Verkippung des Scanngebiets, synchronisiert mit der ersten Scannvorrichtung und/oder der zweiten Scannvorrichtung, eine einstellbare Divergenz des Laserstrahls zu erzeugen. Der Schaltkreis ist zudem eingerichtet, den Divergenzmodulator derart zu steuern, dass der Divergenzmodulator das Scanngebiet mit einem von der Scannbearbeitungslinie abhängigen Verkippungswinkel zur Referenzebene verkippt, so dass das Scanngebiet zur Gewebevolumenbearbeitung im Augengewebe mit einer von der Scannbearbeitungslinie abhängigen Verkippung zur Referenzebene entlang der Scannbearbeitungslinie verfahren wird.

In einer Ausführungsvariante ist der Schaltkreis eingerichtet, das Scannersystem derart zu steuern, dass das Scannersystem das durch die erste zusätzliche Scannbewegungskomponente und die zweite zusätzliche Scannbewegungskomponente definierte Scanngebiet entlang der Scannbearbeitungslinie verfährt, welche auf einer Bearbeitungsfläche innerhalb eines im Auge zu bearbeitenden Gewebevolumens verläuft. Der Schaltkreis ist weiter eingerichtet, den Divergenzmodulator derart zu steuern, dass der Divergenzmodulator das Scanngebiet mit einem von der Scannbearbeitungslinie abhängigen Verkippungswinkel derart verkippt, dass beim Verfahren des Scanngebiets entlang der Scannbearbeitungslinie ein erster Randbereich des Scanngebiets entlang einer oberen Aussenfläche des Gewebevolumens geführt wird, die einer Hornhautoberfläche zugewandt ist, und ein dem ersten Randbereich gegenüberliegender zweiter Randbereich des Scanngebiets entlang einer unteren Aussenfläche des Gewebevolumens geführt wird, die von der Hornhautoberfläche abgewandt ist. Die sich mit dem Verfahren auf der Bearbeitungslinie verändernde Verkippung des Scanngebiets, bei welcher dessen Randbereiche Aussenflächen des zu bearbeitenden Gewebevolumens abfahren, ermöglicht eine effiziente und flexible Bearbeitung von unterschiedlich geformten Gewebevolumen.

In einer Ausführungsvariante ist der Schaltkreis eingerichtet, das Scannersystem derart zu steuern, dass das durch die erste zusätzliche Scannbewegungskomponente und die zweite zusätzliche Scannbewegungskomponente definierte Scanngebiet entlang der Scannbearbeitungslinie verfahren wird, welche spiralförmig oder spiralarmförmig innerhalb eines im Auge zu bearbeitenden Lentikels verläuft.

In einer Ausführungsvariante ist der Schaltkreis eingerichtet, das Scannersystem derart zu steuern, dass das Scannersystem das durch die erste zusätzliche Scannbewegungskomponente und die zweite zusätzliche Scannbewegungskomponente definierte Scanngebiet entlang der Scannbearbeitungslinie verfährt, welche entlang Meridianen auf einer Bearbeitungsfläche innerhalb eines im Auge zu bearbeitenden Lentikels verläuft.

In einer Ausführungsvariante ist der Schaltkreis eingerichtet, das Scannersystem derart zu steuern, dass das Scannersystem das durch die erste zusätzliche Scannbewegungskomponente und die zweite zusätzliche Scannbewegungskomponente definierte Scanngebiet entlang der Scannbearbeitungslinie verfährt, welche entlang einer Zentrumslinie innerhalb eines im Auge zu bearbeitenden Tunnels verläuft.

In einer Ausführungsvariante umfasst die ophthalmologische Vorrichtung einen Scannlängenmodulator, welcher eingerichtet ist, eine Länge der zweiten zusätzlichen Scannbewegungskomponente in der zweiten Scannrichtung zu verändern, um eine Breite des durch die erste zusätzliche Scannbewegungskomponente und die zweite zusätzliche Scannbewegungskomponente definierten Scanngebiets einzustellen, und der Schaltkreis ist eingerichtet, den Scannlängenmodulator derart zu steuern, dass der Scannlängenmodulator die Breite des Scanngebiets abhängig von der Scannbearbeitungslinie einstellt, so dass das Scanngebiet zur Gewebevolumenbearbeitung im Augengewebe mit einer von der Scannbearbeitungslinie abhängigen Breite entlang der Scannbearbeitungslinie verfahren wird.

### Kurze Beschreibung der Zeichnungen

Nachfolgend wird eine Ausführung der vorliegenden Erfindung anhand eines Beispiels beschrieben. Das Beispiel der Ausführung wird durch die folgenden beigelegten Figuren illustriert:
- Figur 1:: zeigt ein Blockdiagramm einer ophthalmologischen Vorrichtung zum Bearbeiten von Augengewebe mittels eines gepulsten Laserstrahls, welche ein Scannersystem zum Lenken des Laserstrahls entlang einer Scannbearbeitungslinie sowie zwei dem Scannersystem vorgeschaltete, wesentlich schnellere Scannvorrichtungen zur Erzeugung zusätzlicher Scannbewegungskomponenten umfasst.
- Figur 2:: zeigt schematisch im Querschnitt ein zu bearbeitendes Gewebevolumen im Augengewebe sowie einen gepulsten Laserstrahl bei der Bearbeitung eines Scanngebiets an zwei verschiedenen Stellen auf einer Bearbeitungslinie mit unterschiedlichem Verkippungswinkel, sowie jeweils eine Aufsicht auf das betreffende Scanngebiet, das durch zusätzliche Scannbewegungskomponenten zur Bearbeitungslinie definiert wird.
- Figur 3:: zeigt einen schematischen Querschnitt eines Abschnitts des Laserstrahls in einem Divergenzmodulator mit mindestens einer verschiebbaren Linse und illustriert die durch die Verschiebung der Linse veränderte Divergenz des Laserstrahls.
- Figur 4:: zeigt eine schematische Aufsicht eines im Augengewebe zu bearbeitenden lentikelförmigen Gewebevolumens mit rundem Umriss, welches mit einem Scanngebiet bearbeitet wird, das entlang mehrerer Bearbeitungslinien verfahren wird, die entlang Meridianen des Gewebevolumens verlaufen.
- Figur 5:: zeigt eine schematische Aufsicht eines zu bearbeitenden lentikelförmigen Gewebevolumens mit elliptischem Umriss im Augengewebe, welches mit einem Scanngebiet bearbeitet wird, das entlang mehrerer Bearbeitungslinien verfahren wird, die entlang Meridianen des Gewebevolumens verlaufen.
- Figur 6:: zeigt eine schematische Aufsicht eines zu bearbeitenden lentikelförmigen Gewebevolumens im Augengewebe, welches mit einem Scanngebiet bearbeitet wird, das entlang einer spiralförmigen Bearbeitungslinie verfahren wird, die quer zu Meridianen des Lentikels verläuft.
- Figur 7:: zeigt eine schematische Aufsicht eines zu bearbeitenden lentikelförmigen Gewebevolumens im Augengewebe, welches mit einem Scanngebiet bearbeitet wird, das entlang mehrerer spiralarmförmigen Bearbeitungslinie verfahren wird, die quer zu Meridianen des Lentikels verlaufen.
- Figur 8:: zeigt eine schematische dreidimensionale Ansicht eines zu bearbeitenden pfostenförmigen Gewebevolumens im Augengewebe, welches mit einem Scanngebiet bearbeitet wird, das entlang einer Bearbeitungslinie verfahren wird, die entlang der vertikalen Zentrumslinie des Pfostens verläuft.
- Figur 9:: zeigt eine schematische dreidimensionale Ansicht eines zu bearbeitenden tunnelförmigen Gewebevolumens im Augengewebe, welches mit einem Scanngebiet bearbeitet wird, das entlang einer Bearbeitungslinie verfahren wird, die entlang der Zentrumslinie des Tunnels verläuft.
- Figur 10:: zeigt schematisch im Querschnitt ein Gewebevolumen im Augengewebe, welches eine variierende Dicke aufweist und mittels eines Scanngebiets bearbeitet wird, das tangential zu einer spiralförmigen Bearbeitungslinie entlang der spiralförmigen Bearbeitungslinie verfahren wird.

### Wege zur Ausführung der Erfindung

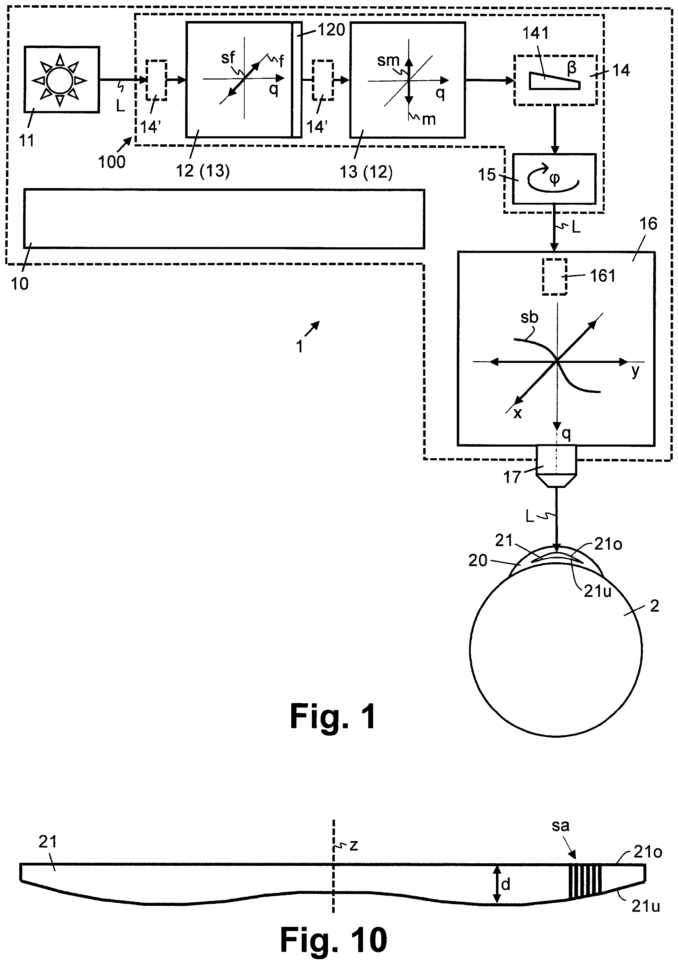

In der Figur 1 bezieht sich das Bezugszeichen 1 auf eine ophthalmologische Vorrichtung zum Bearbeiten von Augengewebe 20 mittels Laserpulsen. Ein Auge 2 ist schematisch im Querschnitt illustriert. Wie in der Figur 1 schematisch dargestellt ist, ist die ophthalmologische Vorrichtung 1 eingerichtet, im zu bearbeitenden Augengewebe 20, insbesondere in der Cornea aber auch in anderem Augengewebe wie Glaskörper oder Linse des Auges 2, ein Gewebevolumen 21 zu bearbeiten, das eine obere Aussenfläche 21o und eine untere Aussenfläche 21u aufweist. Die obere Aussenfläche 21o ist der externen Oberfläche der Cornea 20 respektive der ophthalmologischen Vorrichtung 1 zugewandt. Die untere Aussenfläche 21u ist von der externen Oberfläche der Cornea 20 respektive der ophthalmologischen Vorrichtung 1 abgewandt.

Wie in der Figur 1 schematisch illustriert ist, umfasst die ophthalmologische Vorrichtung 1 eine Laserquelle 11. Die Laserquelle 11 ist eingerichtet einen gepulsten Laserstrahl L zu erzeugen. In einer Ausführungsvariante ist die Laserquelle 11 eingerichtet einen Laserstrahl L mit Femtosekundenlaserpulsen zu erzeugen.

Die ophthalmologische Vorrichtung 1 umfasst überdies ein optisches Übertragungssystem 100, ein Scannersystem 16 und eine Fokussieroptik 17.

Die Fokussieroptik 17 ist eingerichtet, den Laserstrahl L ins Augengewebe 20 zu fokussieren. Die Fokussieroptik 17 umfasst eine oder mehrere optische Linsen. Zur Einstellung der Brennweite (Fokus) umfasst die Fokussieroptik 17 mindestens eine bewegliche Linse. In einer Ausführungsvariante umfasst die Fokussieroptik 17 zudem einen oder mehrere Antriebe, z.B. Elektromotoren, zur automatisierten Bewegung der beweglichen Linse(n) und der dadurch bewirkten Einstellung und Anpassung des Fokus respektive der abtast- und bearbeitbaren Fokalfläche(n) ("Tiefeneinstellung").

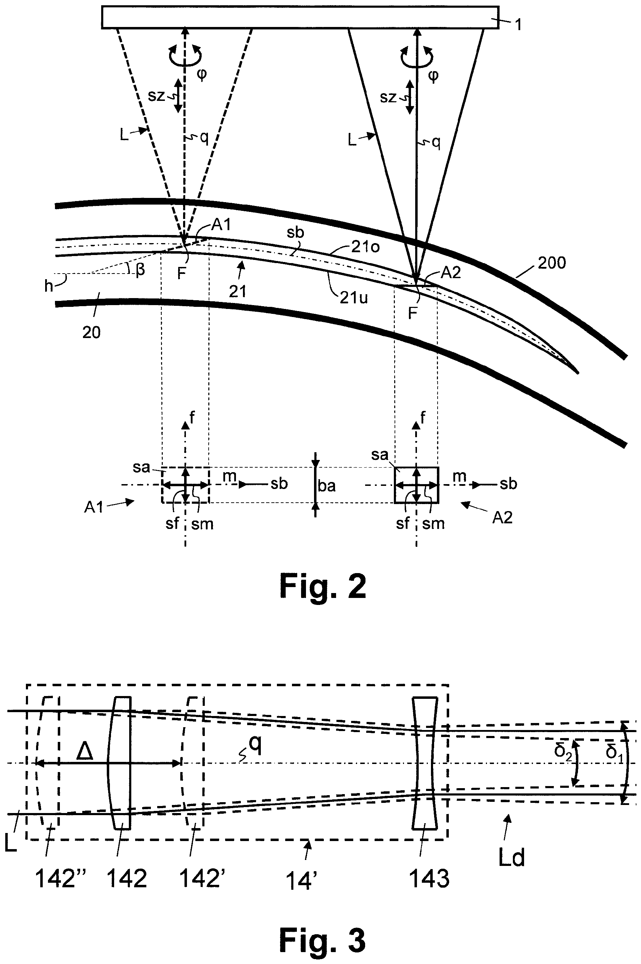

Der gepulste Laserstrahl L wird von der Laserquelle 11 über das nachfolgend beschriebene optische Übertragungssystem 100 dem Scannersystem 16 zugeführt. Das Scannersystem 16 ist eingerichtet, den Laserstrahl L über die Fokussieroptik 17 ins Augengewebe 20 einzustrahlen und im Augengewebe 20 gemäss einem vorgegebenen x/y-Scannmuster entlang einer Scannbearbeitungslinie sb zu lenken (siehe Figuren 2 und 4-9). In einer Ausführungsvariante umfasst das Scannersystem 16 ein Antriebssystem, das mit der Fokussieroptik 17 gekoppelt ist und eingerichtet ist, die Fokussieroptik 17 in x/y-Bearbeitungsrichtungen einer Bearbeitungsebene zu verfahren, welche normal zur optischen Achse q der Fokussieroptik 17 angeordnet ist. In einer Ausführungsvariante umfasst das Scannersystem 16 ein Antriebssystem zur automatisierten Bewegung der Fokussieroptik 17, in Richtung der optischen Achse q der Fokussieroptik 17, oder der beweglichen Linse(n) der Fokussieroptik 17, oder eine zusätzliche Fokussiervorrichtung 161 um den Laserstrahl L im Augengewebe 20 mit einer in Projektionsrichtung verlaufenden Richtungskomponente sz, also in Richtung der optischen Achse q der Fokussieroptik 17, entlang der Scannbearbeitungslinie sb zu lenken, also entlang einer Scannbearbeitungslinie sb mit einer vertikalen Richtungskomponente (z-Richtung) zusätzlich zu den oder anstelle der x/y-Bearbeitungsrichtungen. In einer weiteren Ausführungsvariante ist die zusätzliche Fokussiervorrichtung 161 Teil des optischen Übertragungssystems 100 - kurzum, die zusätzliche Fokussiervorrichtung 161 ist im Strahlengang zwischen der Laserquelle 11 und der Fokussieroptik 17 angeordnet. In einer Ausführungsvariante umfasst das Scannersystem 16 als Alternative oder zusätzlich zu einem oder mehreren Antriebssystemen eines oder mehrere strahlablenkende x/y-Scannermodule, z.B. Scannermodule mit beweglichen Spiegeln, die um eine oder zwei Scannachsen schwenkbar sind, oder optische Modulatoren. In einer Variante ist der Fokussieroptik 17 ein strahlablenkendes Scannersystem mit einem oder mehreren beweglichen (drehbaren) Spiegeln vorgelagert.

Wie in der Figur 1 schematisch illustriert ist, umfasst das optische Übertragungssystem 100 zwei kaskadierte Scannvorrichtungen 12, 13, die dem Scannersystem 16 vorgeschaltet sind ("upstream"). In der Figur 1 soll mit den in Klammern angegebenen Bezugszeichen (12), (13) angedeutet werden, dass in unterschiedlichen Ausführungsvarianten entweder die Scannvorrichtung 12 der Scannvorrichtung 13 vorgeschaltet ist oder umgekehrt. Beide Scannvorrichtungen 12, 13 sind eingerichtet, zur Erzeugung zusätzlicher, der Scannbearbeitungslinie sb überlagerten Scannbewegungskomponenten sf, sm, den gepulsten Laserstrahl L in zwei weitere zueinander abgewinkelte Scannrichtungen f, m abzulenken, mit einer wesentlich höheren Scanngeschwindigkeit, im Vergleich zu der Bearbeitungsgeschwindigkeit, mit der das nachgeschaltete Scannersystem 16 den gepulsten Laserstrahl L im Augengewebe 20 entlang der Scannbearbeitungslinie sb lenkt. Dabei ist die Scannvorrichtung 12 eingerichtet, den gepulsten Laserstrahl L zur Erzeugung der zusätzlichen Scannbewegungskomponente sf mit einer wesentlich höheren Scanngeschwindigkeit in die Scannrichtung f abzulenken, verglichen mit der Scanngeschwindigkeit, mit welcher die Scannvorrichtung 13 den gepulsten Laserstrahl L zur Erzeugung der zusätzlichen Scannbewegungskomponente sm in die Scannrichtung m ablenkt, so dass die zusätzliche Scannbewegungskomponente sf der zusätzlichen Scannbewegungskomponente sm überlagert wird. Die Scannvorrichtungen 12, 13 umfassen beispielsweise Galvano-, Piezo-, Polygon- oder Resonanz-Scannermodule, oder AOM- (Acusto-optischer Modulator) oder EOM- Scannermodul (Electro-optischer Modulator).

Wie in der Figur 2 mittels der schematisch dargestellten Aufsichten A1, A2 illustriert wird, wird durch die Überlagerung der durch die Scannvorrichtung 12 erzeugten zusätzlichen Scannbewegungskomponente sf und der durch die Scannvorrichtung 13 erzeugten zusätzlichen Scannbewegungskomponente sm ein Scanngebiet sa definiert, welches durch die Scannvorrichtungen 12, 13 mit wesentlich höheren Scanngeschwindigkeiten mit dem gepulsten Laserstrahl L bearbeitet wird, als die Bearbeitungsgeschwindigkeit, mit der das Scannersystem 16 den gepulsten Laserstrahl L im Augengewebe 20 entlang der Scannbearbeitungslinie sb lenkt.

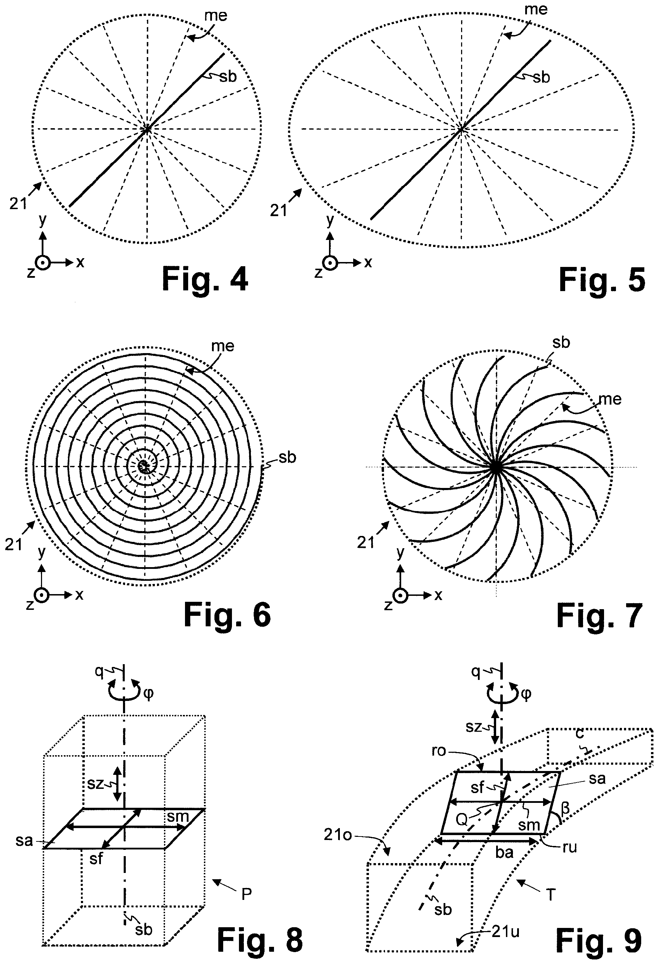

Wie in der Figur 2 schematisch im Querschnitt und in den Figuren 8, 9 in dreidimensionaler Darstellung illustriert ist, wird das durch die zusätzlichen Scannbewegungskomponente sf, sm erzeugte Scanngebiet sa durch das Scannersystem 16 im Augengewebe 20 entlang der Scannbearbeitungslinie sb verfahren, wodurch ein Gewebebearbeitung im Augengewebe 20 bewirkt wird. In einer Ausführungsvariante, sind die Scannvorrichtungen 12, 13 und das Scannersystem 16 derart eingerichtet und angeordnet, dass ihre Scannrichtungen quer zueinander verlaufen und das durch die zusätzlichen Scannbewegungskomponenten sf, sm erzeugte Scanngebiet sa und die Bearbeitungslinie sb quer zueinander verlaufen, so dass beim Verfahren des Scanngebiets sa durch das Scannersystem 16 entlang der Scannbearbeitungslinie sb im Augengewebe 20 eine (dreidimensionale) Volumenbearbeitung bewirkt wird, wie beispielsweise in der Figur 8 illustriert wird.

Im Beispiel der Figur 8 wird das durch die zusätzlichen Scannbewegungskomponenten sf, sm erzeugte Scanngebiet sa mittels der Fokussiervorrichtung 161 und/oder der Fokussieroptik 17 entlang einer normal dazu verlaufenden Bearbeitungslinie sb, mit einer in Projektionsrichtung verlaufenden Richtungskomponente sz, also in Richtung der optischen Achse q der Fokussieroptik 17 verfahren, z.B., um Abschattungen zu vermeiden, von unten - weiter von der äusseren Augen- oder Cornea-Oberfläche 200 entfernt, nach oben - näher zur äusseren Augen- oder Cornea-Oberfläche 200, wodurch eine Volumenbearbeitung in Form eines Pfostens P oder einer Säule bewirkt wird.

Im Beispiel der Figur 9 wird das durch die zusätzlichen Scannbewegungskomponenten sf, sm erzeugte Scanngebiet sa um einen Verkippungswinkel β bezüglich einer Referenzebene zur Bearbeitungslinie sb verkippt, so dass die zusätzlichen Scannbewegungskomponenten sf, sm und das dadurch definierte und erzeugte Scanngebiet sa quer zur Bearbeitungslinie sb verlaufen, wie im Querschnitt im Beispiel der Figur 2 dargestellt ist. Als Referenzebene dient hierzu eine horizontale, normal zur optischen Achse q der Fokussieroptik 17 verlaufende Referenzebene h oder eine tangential zum aktuellen Arbeitspunkt auf der Bearbeitungslinie sb verlaufende Referenzebene. Das zur Bearbeitungslinie sb verkippte Scanngebiet sa wird durch das Scannersystem 16 entlang der Bearbeitungslinie sb verfahren, wodurch im Augengewebe 20 eine Volumenbearbeitung in Form eines Tunnels T bewirkt wird.

Zur Verkippung des durch die zusätzlichen Scannbewegungskomponenten sf, sm definierten Scanngebiets sa umfasst die ophthalmologische Vorrichtung 1 respektive das optische Übertragungssystem 100 einen Divergenzmodulator 14, 14'.

Wie in der Figur 1 schematisch dargestellt ist, umfasst der Divergenzmodulator 14 in einer Ausführungsvariante ein optisches Element 141, das im Strahlengang eingekoppelt den Scannvorrichtungen 12, 13 nachgeschaltet und dem Scannersystem 16 vorgeschaltet ist. Das optische Element 141 ist eingerichtet, eine von den zusätzlichen Scannbewegungskomponenten sf, sm abhängige Divergenz des Laserstrahls L zu erzeugen und dadurch das Scanngebiet sa zu verkippen. Das optische Element 141 umfasst eine Keilplatte, ein Prisma, eine Linse, ein diffraktives optisches Element und/oder einen asphärischen Spiegel. In einer Ausführungsvariante ist das das optische Element 141 um eine optische Übertragungsachse q drehbar ausgebildet, wodurch die Verkippung des Scanngebiets sa zusätzlich durch eine Verdrehung um die Übertragungsachse q veränderbar und damit einstellbar ist. In einer Variante wird das optische Element transversal zur optischen Achse q der Fokussieroptik 17 verschoben.

Wie in der Figur 1 angedeutet ist, ist der Divergenzmodulator 14' in alternativen Ausführungen der Scannvorrichtung 12 oder 13 vorgeschaltet in den Strahlengang eingekoppelt und eingerichtet, die Divergenz des ihm zugeführten gepulsten Laserstrahls L dynamisch zu verändern und dadurch zumindest eine der Scannbewegungskomponenten sf, sm und damit das Scanngebiet sa zu verkippen.

Die Figur 3 illustriert schematisch eine Ausführungsvariante des Divergenzmodulators 14' mit zwei seriell angeordneten optischen Linsen 142, 143, von denen mindestens eine zur Modulation der Divergenz des gepulsten Laserstrahls L auf der optischen Übertragungsachse q verschiebbar ist. Zur dynamischen Modulation der Divergenz des gepulsten Laserstrahls L ist die bewegliche Linse 142 mit einem Bewegungstreiber gekoppelt. Wie im Beispiel der Figur 3 ersichtlich ist, weist der gepulste Laserstrahl L bei einer ersten Grundstellung 142' der beweglichen Linse eine entsprechende Divergenz δ₁ auf. Bei einer Verschiebung der beweglichen Linse 142 auf der Übertragungsachse q verändert sich die Divergenz des zugeführten Laserstrahls L kontinuierlich und hat bei der um die Auslenkungsdistanz Δ verschobenen Stellung 142" eine veränderte Divergenz δ₂.

Wie in der Figur 1 schematisch dargestellt ist, umfasst die ophthalmologische Vorrichtung 1 ein Rotationselement 15, das im Strahlengang den Scannvorrichtungen 12, 13 nachgeschaltet ist. Das Rotationselement 15 ist eingerichtet, das durch die zusätzlichen Scannbewegungskomponenten sf, sm definierte Scanngebiet sa um einen Drehwinkel ϕ um die optische Übertragungsachse q zu drehen, so dass ein um den Drehwinkel ϕ rotiertes Scanngebiet sa definiert wird, wie in der Figur 9 schematisch dargestellt ist. In einer Ausführungsvariante umfasst das Rotationselement 15 einen K-Spiegel oder ein Prisma zum Drehen des Scanngebiets sa. Der Drehwinkel ϕ wird beispielsweise abhängig von der aktuellen Position auf der Bearbeitungslinie sb dynamisch eingestellt und verändert, so dass das Scanngebiet sa mit einem definierten Sollwinkel zur Bearbeitungslinie sb ausgerichtet ist.

In der Figur 1 bezeichnet das Bezugszeichen 120 einen Scannlängenmodulator, welcher eingerichtet ist, eine Länge der zusätzlichen Scannbewegungskomponente sf in der Scannrichtung f zu verändern, um eine bestimmte Breite ba des durch die zusätzlichen Scannbewegungskomponenten sf, sm definierten Scanngebiets sa einzustellen (siehe Figur 2). In einer Ausführungsvariante ist der Scannlängenmodulator 120 im Strahlengang der Scannvorrichtung 12 nachgeschaltet und umfasst einstellbare Blenden (Shutter), welche die Länge der Scannbewegungskomponente sf in der Scannrichtung f begrenzen, um die gewünschte Breite ba des Scanngebiets sa zu erreichen. In einer alternativen Ausführung ist der Scannlängenmodulator 120 elektronisch ausgeführt und umfasst eine Steuerleitung von der Scannvorrichtung 12, über welche ein Zeilensignal der Scannvorrichtung 12 einem Shutter zugeführt, welcher den von der Laserquelle 11 erzeugten Laserstrahl L synchronisiert mit dem Zeilensignal so moduliert, dass die gewünschte Breite ba des Scanngebiets sa erreicht wird. Mit einem elektronischen Shutter lassen sich auch Randbereiche von beliebig gekrümmten und/oder geneigten Scanngebieten sa, die ohne Shutter über eine gewünschte Scannbreite ba hinausgehen, auf die gewünschte Scannbreite ba begrenzen. Alternativ oder zusätzlich zum Scannlängenmodulator 120 ist in weiteren Ausführungen ein weiterer Scannlängenmodulator zur Begrenzung der Scannbreite (respektive Scannhöhe) in der Scannrichtung m vorgesehen. In einer weiteren Ausführungsvariante ist ein zweidimensionaler Scannlängenmodulator zur Begrenzung des Scangebiet sa vor dem Divergenzmodulator 14 vorgesehen. Ein solcher zweidimensionaler Scannlängenmodulator, z.B. in Form einer schaltbaren Maske oder Blende, ermöglicht eine Formanpassung des Scanngebiets sa, z.B. eine hexagonale oder rautenförmige Formanpassung respektive Begrenzung des Scanngebiets sa. Hexagonale Begrenzungen ermöglichen im Gegensatz zu kreisrunden eine vollständige Füllung von Volumina. Rautenförmige Scanngebiete sa ermöglichen alternativ zu nicht orthogonalen Scannachsen respektive zu entsprechenden nicht orthogonalen Scannbewegungskomponenten sf und sm eine abschattungsfreie Bearbeitung von Volumen mit aneinanderliegenden Scanngebieten sa.

Wie in der Figur 1 schematisch dargestellt ist, umfasst die ophthalmologische Vorrichtung 1 einen elektronischen Schaltkreis 10 zur Steuerung der Laserquelle 11, des optischen Übertragungssystems 100 und dessen Module, einschliesslich der Scannvorrichtungen 12, 13, des Divergenzmodulators 14, 14', des Rotationselements 15 und des Scannlängenmodulators 120 , sowie des Scannersystems 16, der Fokussiervorrichtung 161 und/oder Fokussieroptik 17 und von Antrieben dieser Module, Komponenten und Elemente. Der Schaltkreis 10 realisiert eine programmierbare Steuervorrichtung und umfasst beispielsweise einen oder mehrere Prozessoren mit Programm- und Datenspeicher sowie programmierte Softwaremodule zur Steuerung der Prozessoren, und/oder andere programmierbare Schaltungen oder Logikeinheiten wie ASICs (Application Specific Integrated Circuit). Zur Steuerung der verschiedenen Komponenten und Module der ophthalmologischen Vorrichtung 1 erzeugt der Schaltkreis 10 entsprechende Steuersignale für diese Komponenten und Module.

Der Schaltkreis 10 ist eingerichtet, die ophthalmologische Vorrichtung 1 gemäss gespeicherten Behandlungsdaten derart zu steuern, dass sie im Augengewebe 20 eine durch die Behandlungsdaten definierte Gewebevolumenbearbeitung durchführt. Dazu aktiviert der Schaltkreis die Laserquelle 11 und steuert die Scannvorrichtungen 12, 13 so an, dass diese den gepulsten Laserstrahl L von der Laserquelle 11 in zwei abgewinkelt zu einander verlaufenden Scannrichtungen f, m ablenken, welche zwei einander überlagerte Scannbewegungskomponenten sf, sm bestimmen und damit ein Scanngebiet sa definieren, das durch den gepulsten Laserstrahl L bearbeitet wird. Der Schaltkreis 10 steuert zudem das Scannersystem 16 so, dass dieses das durch die Scannbewegungskomponenten sf, sm definierte und bearbeitete Scanngebiet sa im Augengewebe 20 entlang einer durch die Behandlungsdaten definierten Scannbearbeitungslinie sb verfährt und so die gewünschte Gewebevolumenbearbeitung bewirkt. Die Figuren 1 und 2 illustrieren dazu im Querschnitt ein lentikelförmiges Gewebevolumen 21, mit einer unteren Aussenfläche 21u und einer oberen Aussenfläche 21o, das im Augengewebe 20, insbesondere in der Cornea 20 bearbeitet wird.

An dieser Stelle soll darauf hingewiesen werden, dass mit dem Begriff "Gewebevolumenbearbeitung" neben der Anwendung zum Gewebevolumenabbau im Augengewebe 20 mittels des gepulsten Laserstrahls L auch Behandlungen mitumfasst sind, bei welchen das bearbeitete Gewebevolumen 21, bei entsprechend reduzierter Energie und/oder zeitlicher Begrenzung des eingestrahlten gepulsten Laserstrahls L (durch Steuerung der Laserquelle 11), nicht aufgelöst und dadurch abgebaut, sondern mittels des gepulsten Laserstrahls L (therapeutisch) bestrahlt wird. Solche therapeutisch bestrahlenden Anwendungen der Gewebevolumenbearbeitung umfassen die Bestrahlung des Augengewebes der Augenlinse zum Erhöhen der Elastizität (z.B. durch senkrechte Säulen parallel zur optischen Achse des Auges 2), die Bestrahlung des Augengewebes der Augenlinse zum Reversieren des Grauen Stars (Bleichen oder "Bleaching"), die Bestrahlung des Augengewebes des Glaskörpers des Auges 2 zum Auflösen von sogenannten "Floatern", die Bestrahlung der Cornea 20 oder von Implantaten im Augengewebe zur Änderung des Brechungsindex oder zur Formänderung, die Bestrahlung des Augengewebes der Augenlinse zum Weichmachen der Linse, um diese danach durch Absaugen, ohne Verwendung von Ultraschall-Phakoenergie, zu entfernen. Weitere Anwendungen der Gewebevolumenbearbeitung umfassen das Perforieren von Gewebe zu Erhöhung der Diffusion und die Erhöhung der Gewebefestigkeit durch Cross-Linking mit Hilfe von Femtosekundenpulsen des gepulsten Laserstrahls L.

Die Figuren 4-7 zeigen in schematischer Aufsicht (mit normal zur x/y-Zeichenebene verlaufender optischen Achse des Auges 2) verschiedene Varianten von Schnittausführungen für die Bearbeitung im Augengewebe 20 eines lentikelförmigen Gewebevolumens 21 durch Verfahren des durch die Scannvorrichtungen 12, 13 erzeugten Scanngebiets sa entlang einer oder mehreren Bearbeitungslinien sb durch das Scannersystem 16. Die Figur 4 zeigt eine Schnittausführung mit mehreren Bearbeitungslinien sb, die entlang Meridianen me eines zu bearbeitenden runden, lentikelförmigen Gewebevolumens 21 verlaufen. Die Figur 5 zeigt eine Schnittausführung mit mehreren Bearbeitungslinien sb, die entlang Meridianen me eines zu bearbeitenden elliptischen, lentikelförmigen Gewebevolumens 21 verlaufen. Die Figur 6 zeigt eine Schnittausführung mit einer spiralförmigen Bearbeitungslinie sb, die quer zu Meridianen me eines zu bearbeitenden runden, lentikelförmigen Gewebevolumens 21 verläuft und ein Spiralzentrum auf der optischen Achse des Auges 2 hat. Ein Beispiel einer Gewebevolumenbearbeitung, bei welcher ein verkipptes Scanngebiet sa tangential zu einer spiralförmigen Bearbeitungslinie sb ausgerichtet und entlang dieser spiralförmigen Bearbeitungslinie sb verfahren wird, ist in Figur 10 schematisch im Querschnitt illustriert und wird später eingehender beschrieben. Die Figur 7 zeigt eine Schnittausführung mit mehreren spiralarmförmigen Bearbeitungslinien sb, die ausgehend vom peripheren Rand eines zu bearbeitenden runden, lentikelförmigen Gewebevolumens 21 auf einen Zentrumspunkt auf der optischen Achse des Auges 2 hinzulaufen (oder umgekehrt) und dabei die Meridiane me schneiden.

Zusätzlich zur Steuerung der Scannvorrichtungen 12, 13 und des Scannersystems 16 gemäss den Behandlungsdaten zum Bearbeiten und Verfahren des Scanngebiets sa entlang der Bearbeitungslinie sb, ist der Schaltkreis 10 überdies eingerichtet, abhängig von den Behandlungsdaten und der jeweiligen (aktuellen) Bearbeitungsposition P des Scannersystems 16 auf der Bearbeitungslinie sb, den Divergenzmodulator 14, 14' zu steuern, um das Scanngebiet sa gezielt und variierbar um einen Verkippungswinkel β bezüglich einer Referenzebene zur Bearbeitungslinie sb zu verkippen; das Rotationselement 15 zu steuern, um das Scanngebiet sa um einen Drehwinkel ϕ um die optische Übertragungsachse q zu drehen und gezielt und variierbar zur Bearbeitungslinie sb auszurichten, beispielsweise so dass das Scanngebiet sa während des Verfahrens relativ zur spiralförmigen oder spiralarmförmigen Bearbeitungslinien sb gleich ausgerichtet bleibt; den Scannlängenmodulator 120 zu steuern, um die Breite ba des Scanngebiets sa gezielt und variierbar auf den Bearbeitungspunkt auf der Bearbeitungslinie sb einzustellen, beispielsweise so dass das Scanngebiets sa abhängig von seiner Lage oder der Position auf der spiralförmigen , spiralarmförmigen oder entlang Meridianen verlaufenden Bearbeitungslinie sb mit unterschiedlicher Breite sb verfahren wird; und/oder die Fokussiervorrichtung 161 und/oder die Fokussieroptik 17 zu steuern, um den Fokus des Laserstrahls L in Projektionsrichtung zu verschieben.

Der Schaltkreis 10 ist somit eingerichtet, die ophthalmologische Vorrichtung 1 respektive deren Module und Komponenten gemäss gespeicherten Behandlungsdaten derart zu steuern, dass sie im Augengewebe 20 durch das Bearbeiten und Verfahren des Scanngebiets sa entlang einer oder mehrerer Bearbeitungslinien sb (siehe z.B. die Bearbeitungslinien sb in Figuren 4-7) eines oder mehrere tunnelförmige Gewebevolumen T (siehe Figur 9) bearbeitet, welche parallel zu einer normal zur optischen Achse des Auges verlaufenden Ebene verlaufen (bei einem liegenden Patienten horizontal), um gezielt eine durch die Behandlungsdaten definierte Volumenbearbeitung im Augengewebe 20 zu erzeugen (siehe z.B. lentikelförmige Gewebevolumen in Figuren 1 und 2). Dabei steuert der Schaltkreis 10 das Scannersystem 16 und die Fokussiervorrichtung 161 respektive Fokussieroptik 17 derart, dass diese das Scanngebiet sa entlang einer Scannbearbeitungslinie sb verfahren, welche entlang einer Zentrumslinie c des zu bearbeitenden Tunnels T verläuft.

Wie in der Figur 2 schematisch im Querschnitt und in der Figur 9 beispielhaft anhand des tunnelförmigen Gewebevolumens respektive Tunnelsegments T dargestellt ist, ist der Schaltkreis 10 eingerichtet, den Divergenzmodulator 14', abhängig vom aktuellen Bearbeitungspunkt Q des Scannersystems 16 auf der Bearbeitungslinie sb, derart zu steuern, respektive der Divergenzmodulator 14 ist so eingestellt, dass das von den Scannvorrichtungen 12, 13 bearbeitete Scanngebiet sa so mit einem Verkippungswinkel β zu einer Referenzebene zur Bearbeitungslinie sb verkippt wird, beispielsweise zu einer horizontal durch den Bearbeitungspunkt Q verlaufenden Referenzebene h, dass ein oberer Randbereich ro des Scanngebiets sa entlang der oberen Aussenfläche 21o des zu bearbeitenden Gewebevolumens 21 geführt wird und ein dem oberen Randbereich ro gegenüberliegender unterer Randbereich ru des Scanngebiets sa entlang der unteren Aussenfläche 21o des zu bearbeitenden Gewebevolumens 21 geführt wird.

Wie in der Figur 2 schematisch in der Aufsicht und in der Figur 9 beispielhaft anhand des tunnelförmigen Gewebevolumens respektive Tunnelsegments T dargestellt ist, ist der Schaltkreis 10 eingerichtet, den Scannlängenmodulator 120 abhängig vom aktuellen Bearbeitungspunkt Q des Scannersystems 16 auf der Bearbeitungslinie sb derart zu steuern, dass das von den Scannvorrichtungen 12, 13 bearbeitete Scanngebiet sa eine Breite ba aufweist, die der durch die Bearbeitungsdaten definierten Breite des tunnelförmigen Gewebevolumens respektive Tunnelsegments T am betreffenden Bearbeitungspunkt Q entspricht.

Wie in der Figur 2 schematisch im Querschnitt und in der Figur 9 beispielhaft anhand des tunnelförmigen Gewebevolumens respektive Tunnelsegments T dargestellt ist, ist der Schaltkreis 10 eingerichtet, das Rotationselement 15, abhängig vom aktuellen Bearbeitungspunkt Q des Scannersystems 16 auf der Bearbeitungslinie sb, derart zu steuern, dass das von den Scannvorrichtungen 12, 13 bearbeitete Scanngebiet sa so um einen Drehwinkel ϕ um die optische Übertragungsachse q gedreht wird, dass das Scanngebiet sa mit einem bestimmten Winkel, z.B. normal, zur Bearbeitungslinie sb ausgerichtet ist.

Wie in der Figur 2 schematisch im Querschnitt und in der Figur 9 beispielhaft anhand des tunnelförmigen Gewebevolumens respektive Tunnelsegments T dargestellt ist, ist der Schaltkreis 10 eingerichtet, die Fokussiervorrichtung 161 respektive die Fokussieroptik 17, derart zu steuern, dass die Bearbeitungslinie sb und damit der aktuelle darauf liegende Bearbeitungspunkt Q des Scannersystems 16 und das daran entlang verfahrene Scanngebiet sa, im Augengewebe 20 in die durch die Behandlungsdaten definierte Tiefe in Projektionsrichtung positioniert wird.

Wie in der Figur 10 schematisch im Querschnitt beispielhaft anhand eines zu bearbeitenden runden Gewebevolumens 21 dargestellt ist, welches eine variierende Dicke d, d.h. einen sich über den Durchmesser des Gewebevolumens 21 ändernden Abstand zwischen seiner unteren Aussenfläche 21u und seiner oberen Aussenfläche 21o aufweist, ist der Schaltkreis 10 eingerichtet, abhängig von den Behandlungsdaten und der jeweiligen (aktuellen) Bearbeitungsposition P des Scannersystems 16 auf einer spiralförmigen Bearbeitungslinie sb (gemäss Figur 6), den Divergenzmodulator 14, 14' so zu steuern, dass er das Scanngebiet sa gezielt so verkippt, dass es parallel zur Zentrumsachse z verläuft; das Rotationselement 15 so zu steuern, dass es das Scanngebiet sa gezielt so um einen Drehwinkel ϕ um die optische Übertragungsachse q dreht, dass das Scanngebiet sa an der aktuellen Bearbeitungsposition P tangential zur spiralförmigen Bearbeitungslinie sb ausgerichtet ist; und den Scannlängenmodulator 120 so zu steuern, dass die Scannbreite(respektive "Scannhöhe") des Scanngebiets sa in der Scannrichtung m gezielt auf die durch die Behandlungsdaten definierte Dicke d des Gewebevolumens 21 an der aktuellen Bearbeitungsposition P eingestellt wird.

## Patentansprüche

1. Ophthalmologische Vorrichtung (1) zum Bearbeiten von Augengewebe (20), umfassend eine Laserquelle (11), die eingerichtet ist, einen gepulsten Laserstrahl (L) zu erzeugen; ein Scannersystem (16), das eingerichtet ist, den gepulsten Laserstrahl (L) mit einer Bearbeitungsgeschwindigkeit im Augengewebe (20) entlang einer Scannbearbeitungslinie (sb) zu lenken, und eine erste dem Scannersystem (16) vorgeschaltete Scannvorrichtung (13), welche eingerichtet ist, den gepulsten Laserstrahl (L) zur Erzeugung einer ersten zusätzlichen Scannbewegungskomponente (sm) in einer ersten Scannrichtung (m) abzulenken mit einer im Vergleich zur Bearbeitungsgeschwindigkeit wesentlich höheren ersten Scanngeschwindigkeit, so dass die erste zusätzliche Scannbewegungskomponente (sm) der Scannbearbeitungslinie (sb) überlagert wird, **gekennzeichnet durch**
eine zweite dem Scannersystem (16) vorgeschaltete Scannvorrichtung (12), welche eingerichtet ist, den gepulsten Laserstrahl (L) zur Erzeugung einer zweiten zusätzlichen Scannbewegungskomponente (sf) in einer zweiten Scannrichtung (f) abzulenken, die abgewinkelt zur ersten Scannrichtung (m) der ersten zusätzlichen Scannbewegungskomponente (sm) verläuft, mit einer im Vergleich zur ersten Scanngeschwindigkeit wesentlich höheren zweiten Scanngeschwindigkeit, so dass die zweite zusätzliche Scannbewegungskomponente (sf) der ersten zusätzlichen Scannbewegungskomponente (sm) überlagert wird und durch die Ablenkung des gepulsten Laserstrahls (L) mit der ersten zusätzlichen Scannbewegungskomponente (sm) in der ersten Scannrichtung (m) und der überlagerten zweiten zusätzlichen Scannbewegungskomponente (sf) in der zweiten Scannrichtung (f) ein Scanngebiet (sa) bearbeitet wird, und
einen Schaltkreis (10), welcher eingerichtet ist, das Scannersystem (16) derart mit der ersten Scannvorrichtung (13) und der zweiten Scannvorrichtung (12) synchronisiert zu steuern, dass das Scanngebiet (sa) durch das Scannersystem (16) entlang der Scannbearbeitungslinie (sb) verfahren wird und im Augengewebe (20) eine Gewebevolumenbearbeitung bewirkt wird.

2. Ophthalmologische Vorrichtung (1) nach Anspruch 1, **dadurch gekennzeichnet, dass** das Scannersystem (16), die erste Scannvorrichtung (13) und die zweite Scannvorrichtung (12) derart ausgeführt sind, dass das durch die erste zusätzliche Scannbewegungskomponente (sm) und die zweite zusätzliche Scannbewegungskomponente (sf) definierte Scanngebiet (sa) und die Bearbeitungslinie (sb) quer zueinander verlaufen, und das Scanngebiet (sa) durch das Scannersystem (16) zur Volumenbearbeitung im Augengewebe (20) entlang der Scannbearbeitungslinie (sb) verfahren wird.

3. Ophthalmologische Vorrichtung (1) nach einem der Ansprüche 1 oder 2, **dadurch gekennzeichnet, dass** das Scannersystem (16) eine Fokussiervorrichtung (161) umfasst und eingerichtet ist, einen Fokus des gepulsten Laserstrahls (L) im Augengewebe (20) mit einer in Projektionsrichtung verlaufenden Richtungskomponente (sz) zu bewegen, um das durch die erste zusätzliche Scannbewegungskomponente (sm) und die zweite zusätzliche Scannbewegungskomponente (sf) definierte Scanngebiet (sa) zur Volumenbearbeitung im Augengewebe (20) mit einer in Projektionsrichtung verlaufenden Richtungskomponente (sz) zu verfahren.

4. Ophthalmologische Vorrichtung (1) nach einem der Ansprüche 1 bis 3, **dadurch gekennzeichnet, dass** die ophthalmologische Vorrichtung (1) ein der ersten Scannvorrichtung (13) und der zweiten Scannvorrichtung (12) nachgeschaltetes Rotationselement (15) umfasst, welches eingerichtet ist, das durch die erste zusätzliche Scannbewegungskomponente (sm) und die zweite zusätzliche Scannbewegungskomponente (sf) definierte Scanngebiet (sa) um eine optische Übertragungsachse (q) zu drehen, und dass der Schaltkreis (10) eingerichtet ist, das Rotationselement (15) derart zu steuern, dass das Rotationselement das Scanngebiet (sa) mit einem von der Scannbearbeitungslinie (sb) abhängigen Drehwinkel (ϕ) um die optische Übertragungsachse (q) dreht, so dass das Scanngebiet (sa) zur Gewebevolumenbearbeitung im Augengewebe (20) mit einer einstellbaren Ausrichtung zur Scannbearbeitungslinie (sb) entlang der Scannbearbeitungslinie (sb) verfahren wird.

5. Ophthalmologische Vorrichtung (1) nach einem der Ansprüche 1 bis 4, **dadurch gekennzeichnet, dass** die ophthalmologische Vorrichtung (1) einen Divergenzmodulator (14, 14') umfasst, welcher eingerichtet ist, das durch die erste zusätzliche Scannbewegungskomponente (sm) und die zweite zusätzliche Scannbewegungskomponente (sf) definierte Scanngebiet (sa) bezüglich einer Referenzebene (x/y) zu verkippen, und dass der Schaltkreis (10) eingerichtet ist, den Divergenzmodulator (14) derart zu steuern, dass der Divergenzmodulator (14) das Scanngebiet (sa) mit einem einstellbaren Verkippungswinkel (β) zur Referenzebene (x/y) verkippt, so dass das Scanngebiet (sa) zur Gewebevolumenbearbeitung im Augengewebe (20) mit dem einstellbaren Verkippungswinkel (β) zur Referenzebene (x/y) entlang der Scannbearbeitungslinie (sb) verfahren wird.

6. Ophthalmologische Vorrichtung (1) nach Anspruch 5, **dadurch gekennzeichnet, dass** der Divergenzmodulator (14) mindestens ein optisches Element (141) umfasst, welches der ersten Scannvorrichtung (13) und der zweiten Scannvorrichtung (12) nachgeschaltet ist und welches eingerichtet ist, zur Verkippung des Scanngebiets (sa) eine von der ersten zusätzlichen Scannbewegungskomponente (sm) und der zweiten zusätzlichen Scannbewegungskomponente (sf) abhängige Divergenz des Laserstrahls (L) zu erzeugen.

7. Ophthalmologische Vorrichtung (1) nach Anspruch 6, **dadurch gekennzeichnet, dass** das optische Element (141) um eine optische Übertragungsachse (q) drehbar ausgebildet ist.

8. Ophthalmologische Vorrichtung (1) nach einem der Ansprüche 6 oder 7, **dadurch gekennzeichnet, dass** das optische Element (141) mindestens eines aus der folgenden Liste umfasst: Keilplatte, Prisma, Linse, diffraktives optisches Element und asphärischer Spiegel.

9. Ophthalmologische Vorrichtung (1) nach Anspruch 5, **dadurch gekennzeichnet, dass** der Divergenzmodulator (14') der ersten Scannvorrichtung (13) vorgeschaltet ist und eingerichtet ist, zur Verkippung des Scanngebiets (sa), synchronisiert mit mindestens einem aus: der ersten Scannvorrichtung (13) und der zweiten Scannvorrichtung (12), eine einstellbare Divergenz des Laserstrahls (L) zu erzeugen, und dass der Schaltkreis (10) eingerichtet ist, den Divergenzmodulator (14') derart zu steuern, dass der Divergenzmodulator (14') das Scanngebiet (sa) mit einem von der Scannbearbeitungslinie (sb) abhängigen Verkippungswinkel (β) zur Referenzebene (x/y) verkippt, so dass das Scanngebiet (sa) zur Gewebevolumenbearbeitung im Augengewebe (20) mit einer von der Scannbearbeitungslinie (sb) abhängigen Verkippung zur Referenzebene (x/y) entlang der Scannbearbeitungslinie (sb) verfahren wird.

10. Ophthalmologische Vorrichtung (1) nach Anspruch 9, **dadurch gekennzeichnet, dass** der Schaltkreis (10) eingerichtet ist, das Scannersystem (16) derart zu steuern, dass das Scannersystem (16) das durch die erste zusätzliche Scannbewegungskomponente (sm) und die zweite zusätzliche Scannbewegungskomponente (sf) definierte Scanngebiet (sa) entlang der Scannbearbeitungslinie (sb) verfährt, welche auf einer Bearbeitungsfläche innerhalb eines im Auge (2) zu bearbeitenden Gewebevolumens (21) verläuft, und dass der Schaltkreis (10) eingerichtet ist, den Divergenzmodulator (14, 14') derart zu steuern, dass der Divergenzmodulator (14, 14') das Scanngebiet (sa) mit einem von der Scannbearbeitungslinie (sb) abhängigen Verkippungswinkel (β) derart verkippt, dass beim Verfahren des Scanngebiets (sa) entlang der Scannbearbeitungslinie (sb) ein erster Randbereich des Scanngebiets (sa) entlang einer oberen Aussenfläche (21o) des Gewebevolumens (21) geführt wird, die einer Hornhautoberfläche zugewandt ist, und ein dem ersten Randbereich gegenüberliegender zweiter Randbereich des Scanngebiets (sa) entlang einer unteren Aussenfläche (21o) des Gewebevolumens (21) geführt wird, die von der Hornhautoberfläche abgewandt ist.

11. Ophthalmologische Vorrichtung (1) nach einem der Ansprüche 1 bis 10, **dadurch gekennzeichnet, dass** der Schaltkreis (10) eingerichtet ist, das Scannersystem (16) derart zu steuern, dass das Scannersystem (16) das durch die erste zusätzliche Scannbewegungskomponente (sm) und die zweite zusätzliche Scannbewegungskomponente (sf) definierte Scanngebiet (sa) entlang der Scannbearbeitungslinie (sb) verfährt, welche spiralförmig oder spiralarmförmig innerhalb eines im Auge (2) zu bearbeitenden Lentikels (21) verläuft.

12. Ophthalmologische Vorrichtung (1) nach einem der Ansprüche 1 bis 11, **dadurch gekennzeichnet, dass** der Schaltkreis (10) eingerichtet ist, das Scannersystem (16) derart zu steuern, dass das Scannersystem (16) das durch die erste zusätzliche Scannbewegungskomponente (sm) und die zweite zusätzliche Scannbewegungskomponente (sf) definierte Scanngebiet (sa) entlang der Scannbearbeitungslinie (sb) verfährt, welche entlang Meridianen auf einer Bearbeitungsfläche innerhalb eines im Auge (2) zu bearbeitenden Lentikels (21) verläuft.

13. Ophthalmologische Vorrichtung (1) nach einem der Ansprüche 1 bis 12, **dadurch gekennzeichnet, dass** der Schaltkreis (10) eingerichtet ist, das Scannersystem (16) derart zu steuern, dass das Scannersystem (16) das durch die erste zusätzliche Scannbewegungskomponente (sm) und die zweite zusätzliche Scannbewegungskomponente (sf) definierte Scanngebiet (sa) entlang der Scannbearbeitungslinie (sb) verfährt, welche entlang einer Zentrumslinie innerhalb eines im Auge (2) zu bearbeitenden Tunnels verläuft.

14. Ophthalmologische Vorrichtung (1) nach einem der Ansprüche 1 bis 13, **dadurch gekennzeichnet, dass** die ophthalmologische Vorrichtung (1) einen Scannlängenmodulator (120) umfasst, welcher eingerichtet ist, eine Länge der zweiten zusätzlichen Scannbewegungskomponente (sf) in der zweiten Scannrichtung (f) zu verändern, um eine Breite (ba) des durch die erste zusätzliche Scannbewegungskomponente (sm) und die zweite zusätzliche Scannbewegungskomponente (sf) definierten Scanngebiets (sa) einzustellen, und dass der Schaltkreis (10) eingerichtet ist, den Scannlängenmodulator (120) derart zu steuern, dass der Scannlängenmodulator (120) die Breite (ba) des Scanngebiets (sa) abhängig von der Scannbearbeitungslinie (sb) einstellt, so dass das Scanngebiet (sa) zur Gewebevolumenbearbeitung im Augengewebe (20) mit einer von der Scannbearbeitungslinie (sb) abhängigen Breite (ba) entlang der Scannbearbeitungslinie (sb) verfahren wird.

## Claims

1. Ophthalmological apparatus (1) for treating eye tissue (20), comprising a laser source (11) that is set up to produce a pulsed laser beam (L); a scanner system (16) that is set up to direct the pulsed laser beam (L) at a treatment speed in the eye tissue (20) along a scanning treatment line (sb), and a first scanning apparatus (13) which is connected upstream of the scanner system (16) and is set up to deflect the pulsed laser beam (L), for producing a first additional scanning movement component (sm), in a first scanning direction (m) at a first scanning speed that is considerably higher as compared to the treatment speed, with the result that the first additional scanning movement component (sm) is superposed on the scanning treatment line (sb), **characterized by**
a second scanning apparatus (12) which is connected upstream of the scanner system (16) and is set up to deflect the pulsed laser beam (L), for producing a second additional scanning movement component (sf), in a second scanning direction (f), which is at an angle to the first scanning direction (m) of the first additional scanning movement component (sm), at a second scanning speed that is considerably higher as compared to the first scanning speed, with the result that the second additional scanning movement component (sf) is superposed on the first additional scanning movement component (sm) and a scanning region (sa) is treated by the deflection of the pulsed laser beam (L) with the first additional scanning movement component (sm) in the first scanning direction (m) and the superposed, second additional scanning movement component (sf) in the second scanning direction (f), and
a circuit (10) which is set up to control the scanner system (16) in synchronized fashion with the first scanning apparatus (13) and the second scanning apparatus (12) such that the scanning region (sa) is traveled by the scanner system (16) along the scanning treatment line (sb) and a tissue volume treatment is effected in the eye tissue (20).

2. Ophthalmological apparatus (1) according to Claim 1, **characterized in that** the scanner system (16), the first scanning apparatus (13) and the second scanning apparatus (12) are embodied such that the scanning region (sa), defined by the first additional scanning movement component (sm) and the second additional scanning movement component (sf), and the treatment line (sb) extend transversely with respect to one another, and the scanning region (sa) is travelled along the scanning treatment line (sb) by the scanner system (16) for volume treatment in the eye tissue (20).

3. Ophthalmological apparatus (1) according to one of Claims 1 and 2, **characterized in that** the scanner system (16) comprises a focusing apparatus (161) and is set up to move a focus of the pulsed laser beam (L) in the eye tissue (20) with a directional component (sz) that extends in the projection direction in order to travel the scanning region (sa), defined by the first additional scanning movement component (sm) and the second additional scanning movement component (sf), for volume treatment in the eye tissue (20) with a directional component (sz) that extends in the projection direction.

4. Ophthalmological apparatus (1) according to one of Claims 1 to 3, **characterized in that** the ophthalmological apparatus (1) comprises a rotation element (15), which is connected downstream of the first scanning apparatus (13) and the second scanning apparatus (12) and is set up to rotate the scanning region (sa), defined by the first additional scanning movement component (sm) and the second additional scanning movement component (sf), about an optical transmission axis (q), and **in that** the circuit (10) is set up to control the rotation element (15) such that the rotation element rotates the scanning region (sa) about the optical transmission axis (q) by an angle of rotation (φ) that is dependent on the scanning treatment line (sb), with the result that the scanning region (sa) is travelled along the scanning treatment line (sb) for tissue volume treatment in the eye tissue (20) with a settable orientation with respect to the scanning treatment line (sb).

5. Ophthalmological apparatus (1) according to one of Claims 1 to 4, **characterized in that** the ophthalmological apparatus (1) comprises a divergence modulator (14, 14'), which is set up to tilt the scanning region (sa), defined by the first additional scanning movement component (sm) and the second additional scanning movement component (sf), with respect to a reference plane (x/y), and **in that** the circuit (10) is set up to control the divergence modulator (14) such that the divergence modulator (14) tilts the scanning region (sa) with respect to the reference plane (x/y) at a settable tilt angle (β), with the result that the scanning region (sa) is travelled, for tissue volume treatment in the eye tissue (20), along the scanning treatment line (sb) at the settable tilt angle (β) with respect to the reference plane (x/y).

6. Ophthalmological apparatus (1) according to Claim 5, **characterized in that** the divergence modulator (14) comprises at least one optical element (141) which is connected downstream of the first scanning apparatus (13) and the second scanning apparatus (12) and is set up to produce, for tilting of the scanning region (sa), a divergence of the laser beam (L) that is dependent on the first additional scanning movement component (sm) and the second additional scanning movement component (sf).

7. Ophthalmological apparatus (1) according to Claim 6, **characterized in that** the optical element (141) is configured to be rotatable about an optical transmission axis (q).

8. Ophthalmological apparatus (1) according to one of Claims 6 and 7, **characterized in that** the optical element (141) comprises at least one from the following list: wedge plate, prism, lens, diffractive optical element and aspheric mirror.

9. Ophthalmological apparatus (1) according to Claim 5, **characterized in that** the divergence modulator (14') is connected upstream of the first scanning apparatus (13) and is set up to produce, for tilting the scanning region (sa), synchronized with at least one from: the first scanning apparatus (13) and the second scanning apparatus (12), a settable divergence of the laser beam (L), and **in that** the circuit (10) is set up to control the divergence modulator (14') such that the divergence modulator (14') tilts the scanning region (sa) with respect to the reference plane (x/y) at a tilt angle (β), which is dependent on the scanning treatment line (sb), with the result that the scanning region (sa) is travelled, for tissue volume treatment in the eye tissue (20), along the scanning treatment line (sb) with a tilt, dependent on the scanning treatment line (sb), with respect to the reference plane (x/y).

10. Ophthalmological apparatus (1) according to Claim 9, **characterized in that** the circuit (10) is set up to control the scanner system (16) such that the scanner system (16) travels the scanning region (sa), defined by the first additional scanning movement component (sm) and the second additional scanning movement component (sf), along the scanning treatment line (sb) which extends on a treatment area within a tissue volume (21) that is to be treated in the eye (2), and **in that** the circuit (10) is set up to control the divergence modulator (14, 14') such that the divergence modulator (14, 14') tilts the scanning region (sa) at a tilt angle (β), which depends on the scanning treatment line (sb), such that, during travelling of the scanning region (sa) along the scanning treatment line (sb), a first periphery region of the scanning region (sa) is guided along an upper external area (21o) of the tissue volume (21) which faces a cornea surface, and a second periphery region of the scanning region (sa), situated opposite the first periphery region, is guided along a lower external area (21o) of the tissue volume (21) which faces away from the cornea surface.

11. Ophthalmological apparatus (1) according to one of Claims 1 to 10, **characterized in that** the circuit (10) is set up to control the scanner system (16) such that the scanner system (16) travels the scanning region (sa), defined by the first additional scanning movement component (sm) and the second additional scanning movement component (sf), along the scanning treatment line (sb), which extends in the shape of a spiral or a spiral arm within a lenticule (21) that is to be treated in the eye (2).

12. Ophthalmological apparatus (1) according to one of Claims 1 to 11, **characterized in that** the circuit (10) is set up to control the scanner system (16) such that the scanner system (16) travels the scanning region (sa), defined by the first additional scanning movement component (sm) and the second additional scanning movement component (sf), along the scanning treatment line (sb), which extends along meridians on a treatment area within a lenticule (21) that is to be treated in the eye (2).

13. Ophthalmological apparatus (1) according to one of Claims 1 to 12, **characterized in that** the circuit (10) is set up to control the scanner system (16) such that the scanner system (16) travels the scanning region (sa), defined by the first additional scanning movement component (sm) and the second additional scanning movement component (sf), along the scanning treatment line (sb), which extends along a centre line within a tunnel that is to be treated in the eye (2).

14. Ophthalmological apparatus (1) according to one of Claims 1 to 13, **characterized in that** the ophthalmological apparatus (1) comprises a scanning length modulator (120), which is set up to change a length of the second additional scanning movement component (sf) in the second scanning direction (f) in order to set a width (ba) of the scanning region (sa), defined by the first additional scanning movement component (sm) and the second additional scanning movement component (sf), and **in that** the circuit (10) is set up to control the scanning length modulator (120) such that the scanning length modulator (120) sets the width (ba) of the scanning region (sa) depending on the scanning treatment line (sb), with the result that the scanning region (sa) is travelled, for tissue volume treatment in the eye tissue (20), along the scanning treatment line (sb) with a width (ba) that is dependent on the scanning treatment line (sb) .

## Revendications

1. Dispositif ophtalmologique (1) destiné au traitement de tissus oculaires (20), comprenant une source laser (11) qui est conçue pour produire un rayon laser pulsé (L) ; un système de balayage (16) qui est conçu pour diriger le rayon laser pulsé (L) à une vitesse de traitement dans les tissus oculaires (20) le long d'une ligne de traitement de balayage (sb), et un premier dispositif de balayage (13) placé en amont du système de balayage (16) et conçu pour dévier le rayon laser pulsé (L) afin de produire une première composante de déplacement de balayage supplémentaire (sm) dans une première direction de balayage (m) à une première vitesse de balayage substantiellement supérieure en comparaison avec la vitesse de traitement de sorte que la première composante de déplacement de balayage supplémentaire (sm) est superposée à la ligne de traitement de balayage (sb),
**caractérisé par** un deuxième dispositif de balayage (12) placé en amont du système de balayage (16) et conçu pour dévier le rayon laser pulsé (L) afin de produire une deuxième composante de déplacement de balayage supplémentaire (sf) dans une deuxième direction de balayage (f), qui s'étend selon un angle par rapport à la première direction de balayage (m) du première composante de déplacement de balayage supplémentaire (sm), à une deuxième vitesse de balayage substantiellement supérieure en comparaison avec la première vitesse de balayage de sorte que la deuxième composante de déplacement de balayage supplémentaire (sf) est superposée à la première composante de déplacement de balayage supplémentaire (sm), et la déviation du rayon laser pulsé (L) avec la première composante de déplacement de balayage supplémentaire (sm) dans la première direction de balayage (m) et la deuxième composante de déplacement de balayage supplémentaire (sf) superposée dans la deuxième direction de balayage (f) permet de traiter une zone de balayage (sa), et
un circuit de commutation (10) qui est conçu pour commander de manière synchronisé le système de balayage (16) avec le premier dispositif de balayage (13) et le deuxième dispositif de balayage (12) de telle sorte que la zone de balayage (sa) est traversée par le système de balayage (16) le long de la ligne de traitement de balayage (sb) et un traitement de volume de tissu est provoqué dans les tissus oculaires (20).

2. Dispositif ophtalmologique (1) selon la revendication 1, **caractérisé en ce que** le système de balayage (16), le premier dispositif de balayage (13) et le deuxième dispositif de balayage (12) sont réalisés de telle sorte que la zone de balayage (sa), définie par la première composante de déplacement de balayage supplémentaire (sm) et la deuxième composante de déplacement de balayage supplémentaire (sf), et la ligne de traitement (sb) s'étendent transversalement l'une à l'autre, et la zone de balayage (sa) est traversée par le système de balayage (16) pour le traitement de volume dans les tissus oculaires (20) le long de la ligne de traitement de balayage (sb).

3. Dispositif ophtalmologique (1) selon l'une quelconque des revendications 1 ou 2, **caractérisé en ce que** le système de balayage (16) comprend un dispositif de mise au point (161) et est conçu pour déplacer un foyer du rayon laser pulsé (L) dans les tissus oculaires (20) avec une composante directionnelle (sz) s'étendant dans la direction de projection, afin de traverser la zone de balayage (sa), définie par la première composante de déplacement de balayage supplémentaire (sm) et la deuxième composante de déplacement de balayage supplémentaire (sf), pour le traitement de volume dans les tissus oculaires (20) avec une composante directionnelle (sz) s'étendant dans la direction de projection.

4. Dispositif ophtalmologique (1) selon l'une quelconque des revendications 1 à 3, **caractérisé en ce que** le dispositif ophtalmologique (1) comprend un élément de rotation (15) placé en aval du premier dispositif de balayage (13) et du deuxième dispositif de balayage (12), et qui est conçu pour faire tourner la zone de balayage (sa), définie par la première composante de déplacement de balayage supplémentaire (sm) et la deuxième composante de déplacement de balayage supplémentaire (sf), autour d'un axe de transmission optique (q), et **en ce que** le circuit de commutation (10) est conçu pour commander l'élément de rotation (15) de telle sorte que l'élément de rotation fait tourner la zone de balayage (sa) selon un angle de rotation (ϕ) dépendant de la ligne de traitement de balayage (sb) autour de l'axe de transmission optique (q) de sorte que la zone de balayage (sa) est traversée, pour le traitement de volume de tissu dans les tissus oculaires (20), avec une orientation réglable par rapport à la ligne de traitement de balayage (sb) le long de la ligne de traitement de balayage (sb).

5. Dispositif ophtalmologique (1) selon l'une quelconque des revendications 1 à 4, **caractérisé en ce que** le dispositif ophtalmologique (1) comprend un modulateur de divergence (14, 14') qui est conçu pour faire basculer la zone de balayage (sa), définie par la première composante de déplacement de balayage supplémentaire (sm) et la deuxième composante de déplacement de balayage supplémentaire (sf), par rapport à un plan de référence (x/y), et **en ce que** le circuit de commutation (10) est conçu pour commander le modulateur de divergence (14) de telle sorte que le modulateur de divergence (14) fait basculer la zone de balayage (sa) selon un angle de basculement réglable (β) par rapport au plan de référence (x/y) de sorte que la zone de balayage (sa) est traversée, pour le traitement de volume de tissu dans les tissus oculaires (20), selon l'angle de basculement réglable (β) par rapport au plan de référence (x/y) le long de la ligne de traitement de balayage (sb).

6. Dispositif ophtalmologique (1) selon la revendication 5, **caractérisé en ce que** le modulateur de divergence (14) comprend au moins un élément optique (141) qui est placé en aval du premier dispositif de balayage (13) et du deuxième dispositif de balayage (12) et qui est conçu pour produire une divergence du rayon laser (L) dépendant de la première composante de déplacement de balayage supplémentaire (sm) et de la deuxième composante de déplacement de balayage supplémentaire (sf) afin de faire basculer la zone de balayage (sa).

7. Dispositif ophtalmologique (1) selon la revendication 6, **caractérisé en ce que** l'élément optique (141) est réalisé de manière à ce que l'élément optique (141) puisse tourner autour d'un axe de transmission optique (q).

8. Dispositif ophtalmologique (1) selon l'une quelconque des revendications 6 ou 7, **caractérisé en ce que** l'élément optique (141) comprend au moins un élément de la liste suivante: une plaque cunéiforme, un prisme, une lentille, un élément optique diffractif et un miroir asphérique.

9. Dispositif ophtalmologique (1) selon la revendication 5, **caractérisé en ce que** le modulateur de divergence (14') est placé en amont du premier dispositif de balayage (13) et est conçu, afin de faire basculer la zone de balayage (sa), pour produire une divergence réglable du rayon laser (L) de manière synchronisée avec au moins un parmi : le premier dispositif de balayage (13) et le deuxième dispositif de balayage (12), et **en ce que** le circuit de commutation (10) est conçu pour commander le modulateur de divergence (14') de telle sorte que le modulateur de divergence (14') fait basculer la zone de balayage (sa) selon un angle de basculement (β), dépendant de la ligne de traitement de balayage (sb), par rapport au plan de référence (x/y) de sorte que la zone de balayage (sa) pour le traitement de volume de tissu dans les tissus oculaires (20) est traversée avec un basculement dépendant de la ligne de traitement de balayage (sb) par rapport au plan de référence (x/y) le long de la ligne de traitement de balayage (sb).

10. Dispositif ophtalmologique (1) selon la revendication 9, **caractérisé en ce que** le circuit de commutation (10) est conçu pour commander le système de balayage (16) de telle sorte que le système de balayage (16) traverse la zone de balayage (sa), définie par la première composante de déplacement de balayage supplémentaire (sm) et la deuxième composante de déplacement de balayage supplémentaire (sf), le long de la ligne de traitement de balayage (sb) qui s'étend sur une surface de traitement à l'intérieur d'un volume de tissu (21) à traiter à l'intérieur de l'œil (2), et **en ce que** le circuit de commutation (10) est conçu pour commander le modulateur de divergence (14, 14') de telle sorte que le modulateur de divergence (14, 14') fait basculer la zone de balayage (sa) selon un angle de basculement (β) dépendant de la ligne de traitement de balayage (sb) de telle sorte que lorsque la zone de balayage (sa) est traversée le long de la ligne de traitement de balayage (sb), une première zone marginale de la zone de balayage (sa) est guidée le long d'une surface extérieure supérieure (21o) du volume de tissu (21) qui est tournée vers une surface cornéenne, et une deuxième zone marginale, opposée à la première zone marginale, de la zone de balayage (sa) est guidée le long d'une surface extérieure inférieure (21o) du volume de tissu (21) qui est détournée de la surface cornéenne.

11. Dispositif ophtalmologique (1) selon l'une quelconque des revendications 1 à 10, **caractérisé en ce que** le circuit de commutation (10) est conçu pour commander le système de balayage (16) de telle sorte que le système de balayage (16) traverse la zone de balayage (sa), définie par la première composante de déplacement de balayage supplémentaire (sm) et la deuxième composante de déplacement de balayage supplémentaire (sf), le long de la ligne de traitement de balayage (sb) qui s'étend en spirale ou en forme de bras spiral à l'intérieur d'une lenticule (21) à traiter dans l'œil (2).

12. Dispositif ophtalmologique (1) selon l'une quelconque des revendications 1 à 11, **caractérisé en ce que** le circuit de commutation (10) est conçu pour commander le système de balayage (16) de telle sorte que le système de balayage (16) traverse la zone de balayage (sa), définie par la première composante de déplacement de balayage supplémentaire (sm) et la deuxième composante de déplacement de balayage supplémentaire (sf), le long de la ligne de traitement de balayage (sb) qui s'étend le long de méridiens sur une surface de traitement à l'intérieur d'une lenticule (21) à traiter dans l'œil (2).

13. Dispositif ophtalmologique (1) selon l'une quelconque des revendications 1 à 12, **caractérisé en ce que** le circuit de commutation (10) est conçu pour commander le système de balayage (16) de telle sorte que le système de balayage (16) traverse la zone de balayage (sa), définie par la première composante de déplacement de balayage supplémentaire (sm) et la deuxième composante de déplacement de balayage supplémentaire (sf), le long de la ligne de traitement de balayage (sb) qui s'étend le long d'une ligne centrale à l'intérieur d'un tunnel à traiter dans l'œil (2) .

14. Dispositif ophtalmologique (1) selon l'une quelconque des revendications 1 à 13, **caractérisé en ce que** le dispositif ophtalmologique (1) comprend un modulateur de longueur de balayage (120) qui est conçu pour modifier une longueur de la deuxième composante de déplacement de balayage supplémentaire (sf) dans la deuxième direction de balayage (f) afin de régler une largeur (ba) de la zone de balayage (sa), définie par la première composante de déplacement de balayage supplémentaire (sm) et la deuxième composante de déplacement de balayage supplémentaire (sf), et
**en ce que** le circuit de commutation (10) est conçu pour commander le modulateur de longueur de balayage (120) de telle sorte que le modulateur de longueur de balayage (120) règle la largeur (ba) de la zone de balayage (sa) en fonction de la ligne de traitement de balayage (sb) de sorte que la zone de balayage (sa) pour le traitement de volume de tissu dans les tissus oculaires (20) est traversée sur une largeur (ba) dépendant de la ligne de traitement de balayage (sb) le long de la ligne de traitement de balayage (sb).
